Europäisches Patentamt

European Patent Office

Office européen des brevets

(11) Veröffentlichungsnummer: **0 477 472 B1**

(12) **EUROPÄISCHE PATENTSCHRIFT**

(45) Veröffentlichungstag der Patentschrift: **13.12.95**

(21) Anmeldenummer: **91109691.5**

(22) Anmeldetag: **13.06.91**

Die Akte enthält technische Angaben, die nach dem Eingang der Anmeldung eingereicht wurden und die nicht in dieser Patentschrift enthalten sind.

(51) Int. Cl.6: **C07D 317/38**, C07D 317/36, B01J 31/00

(54) **Verfahren zur Herstellung cyclischer Carbonate**

(30) Priorität: **25.09.90 DE 4030283**

(43) Veröffentlichungstag der Anmeldung:
**01.04.92 Patentblatt 92/14**

(45) Bekanntmachung des Hinweises auf die Patenterteilung:
**13.12.95 Patentblatt 95/50**

(84) Benannte Vertragsstaaten:
**AT BE CH DE DK ES FR GB GR IT LI LU NL SE**

(56) Entgegenhaltungen:
FR-A- 2 517 306
US-A- 2 994 705

PATENT ABSTRACTS OF JAPAN vol. 8, no. 98 (C-221)(1535) 9. Mai 1984 & JP-A- 59 013 776 ( MITSUBISHI ) 24. Januar 1984

CHEMICAL ABSTRACTS, , no. 77, 1972, Columbus, Ohio, US; abstract no. 139417Z. MITSUI: "Alkylene Carbonates". Seite 392

(73) Patentinhaber: **RÜTGERSWERKE AKTIENGE-SELLSCHAFT**
**Mainzer Landstrasse 217**
**D-60326 Frankfurt (DE)**

(72) Erfinder: **Schubert, Frank, Dr.**
**Wiesfurthstrasse 2 a**
**W-4133 Neukirchen-Vluyn (DE)**
Erfinder: **Herzog, Rolf, Dr.**
**Beethovenstrasse 12 a**
**W-4250 Bottrop (DE)**
Erfinder: **Meier, Bert, Dr.**
**Christine-Koch-Strasse 11**
**W-5750 Menden (DE)**
Erfinder: **Zehrfeld, Jürgen, Dr.**
**Elisabethstrasse 32**
**W-4223 Voerde (DE)**

## Beschreibung

Die Erfindung betrifft ein Verfahren zur Herstellung cyclischer Carbonate durch Umsetzung von Glycidylverbindungen mit Kohlendioxid in Gegenwart eines Katalysators.

Urethanharze, die üblicherweise durch Umsetzung von Isocyanatverbindungen hergestellt werden, haben inzwischen aufgrund ihrer vielseitigen Eigenschaften als moderene Werkstoffe eine besondere Bedeutung erlangt.

Anwendungsgebiete sind vor allem der Lack- und Beschichtungssektor, Klebstoffsektor, Gießharzanwendungen und Verbundwerkstoffsektor. Ihre Verwendbarkeit wird jedoch durch die Toxizität der Isocyanate begrenzt.

Mit den durch das erfindungsgemäße Verfahren gewonnenen Reaktionsprodukten können Urethangruppen enthaltende Kunstharze hergestellt werden, ohne daß toxische Substanzen, wie freie Isocyanate oder Phosgene, als Zwischenprodukte verwendet werden müssen.

In der Literatur sind verschiedene Methoden zur Herstellung von 2-Oxo-1,3-dioxolanen, auch cyclische Carbonate genannt, beschrieben.

In WO 84/03701 werden in Alkohol gelöste Epoxide, wie z. B. Propylenoxid gelöst in Methanol, in Gegenwart von Triphenylphosphin als Katalysator mit Kohlendioxid bei etwa 130 °C und einem Überdruck von 21 bar zu cyclischen Carbonaten umgesetzt.

Koodinativ ungesättigte Nickel(O)-Phosphin-Komplexe werden in US 3,748,345 als geeignete Katalysatoren für die Umsetzung von Epoxidverbindungen mit Kohlendioxid verwendet. Beispielsweise wird nach diesem Verfahren Ethylenoxid in benzolischer Lösung in Gegenwart von $Ni(PPh_3)_2$ bei 100 °C und 35 bar in 12 h zu 50 % mit 95 %iger Selektivität zu Ethylencarbonat umgesetzt. Beschrieben sind in dieser Schrift auch die Umsetzungen von 2-Butylenoxid und Epichlorhydrin.

Im Gegensatz zu diesen beiden Verfahren ist die in EP-A 212 409 beschriebene Methode bei Normal- oder bei leicht erhöhtem Durck durchführbar. Als Katalysatoren werden quartäre Ammoniumverbindungen, Amine, Phosphine, Guanidine und Amidine eingesetzt. Zur Durchführung des Verfahrens müssen zusätzliche Cokatalysatoren zugesetzt werden, um die Reaktionsgeschwindigkeit zu erhöhen. Als solche können Halogenide, Carbonate oder Alkali- oder Erdalkalimetalle dienen.

Beispiele für geeignete Katalysatoren sind Triethylammoniumbromid, Benzyltrimethylammoniumcarbonat, Triphenylphosphin, Piperazin, Tetramethylguanidin und Imidazol. Als Cokatalysatoren werden Kalium- und Natriumjodid bevorzugt.

Die Reaktion zwischen der Epoxidverbindung kann direkt oder in Lösung erfolgen. Geeignete Lösungsmittel sind z. B. Toluol, Xylol, Tetrahydrofuran, Dimethylsulfoxid oder Diethylenglycoldimethylether.

Ein typisches Beispiel ist die Umsetzung des Glycidylethers "Beckopox EP 140" mit Kohlendioxid bei Normaldruck und 120 °C in Gegenwart von Triphenylphosphin und Kaliumjodid. In einer Reaktionszeit von 18 h entsteht das 2-Oxo-1,3-dioxolan mit 97,9 %iger Ausbeute und 0,3 % Rest-Epoxidgehalt. Die Reaktionsdauer schwankt zwischen 12 und 29 h je nach Wirksamkeit des Katalysatorsystems.

Bekannt sind weiterhin Verfahren, die in Gegenwart folgender Katalysatoren durchgeführt werden: mit Alkalimetallen behandelter aktivierter Kohlenstoff, Metallhalogenide wie z. B. Magnesium- oder Kalziumhalogenide, Amine, quartäre Ammoniumverbindungen, Ammoniumhalogenide, organische Sulfonsäuresalze, Phosphoniumverbindungen, Hydrazine oder Guanidine.

So werden in US-A-2 994 705 monomere Epoxidverbindungen in Gegenwart von Phosphoniumkatalysatoren zu cyclischen Carbonaten umgesetzt, indem zu Beginn der Reaktion auf -20 °F (~ -27 °C) gekühlt wird und anschließend soviel $CO_2$-Gas in den Reaktionsraum eingeleitet wird, bis ein Überdruck von mehr als 100 p.s.i.g. (6,8 atm) erreicht ist. Letzterer steigt während der Reaktion bei einer Temperatur von 200 - 500 °F (93 - 260 °C) auf das 10- bis 30-fache. Im Anschluß an die Reaktion müssen die Produkte durch Umkristallisieren, Abdestillieren niedrig siedender Verbindungen und / oder durch Destillation bei vermindertem Druck gereinigt werden.

Auch die in CA 77:139417z (JP-A-72 36738), Patent Abst. of Japan vol 8, 1984 (JP-A-84 13776) und FR-A-2 517 303 beschriebenen Umsetzungen von monomeren Epoxyverbindungen mit $Co_2$ in Gegenwart von Phosphoniumverbindungen wie z. B. $MePh_3PJ$ oder $Bu_3P$-MeJ erfolgen in Druckreaktoren nach anfänglicher Kühlung bzw. nach Kühlung durch Zugabe von Trockeneis, das gleichzeitig als Reaktand wirkt, bei erhöhten Temperaturen.

Alle bisher bekannten Verfahren weisen jedoch eine Reihe von Nachteilen auf. Dazu zählen niedrige Reaktionsgeschwindigkeiten, so daß bei erhöhten Temperaturen und Drücken gearbeitet werden muß, geringe Ausbeuten, durch Nebenreaktionen und Katalysatorreste verunreinigte Produkte und bei Einsatz von Hydrazinen die Toxizität und Explosionsgefahr.

EP 0 477 472 B1

Auch das bei Normaldruck durchführbare Verfahren der Schrift EP-A 212 409 das gute Ausbeuten und Selektivitäten aufweist, benötigt lange Reaktionszeiten. Eingesetzte Katalysatoren und Cokatalysatoren müssen aufwendig durch filtrieren oder umkristallisieren entfernt werden, um gute Produktqualitäten und eine heile Farbe zu erhalten.

Aufgabe der Erfindung ist es daher, ein selektives Verfahren zur Herstellung von 2-Oxo-1,3-dioxolanen aus Glycidylverbindungen zur Verfügung zu stellen, durch das bei Normaldruck nach kurzer Reaktionsdauer ein helles Produkt mit hoher Qualität erhalten wird, das keiner weiteren Reinigung bedarf.

Weiterhin ist es Aufgabe der Erfindung, einen löslichen Katalysator zur verfügung zu stellen, der nach Beendigung der Reaktion im Produkt verbleiben kann, ohne dabei zu Verfärbungen oder zu einer Qualitätsminderung des Produkts zu führen.

Die Lösung der Aufgabe erfolgt durch ein Verfahren gemäß Anspruch 1 und seiner besonderen Ausgestaltung gemäß den Ansprüchen 2 bis 7, insbesondere in Gegenwart von Katalysatoren wie sie in den Ansprüchen 6 und 7 genannt sind.

Es wurde gefunden, daß durch Zusatz von quartären Phosphoniumverbindungen die Umsetzung von Glycidylverbindungen mit Kohlendioxid zu 2-Oxo-1,3-dioxolanen bei Normaldruck in erheblich verkürzter Reaktionszeit und mit überraschend hoher Selektivität durchgeführt werden kann.

Katalytisch wirksam sind erfindungsgemäß quartäre Phosphoniumverbindungen der Formel

$$[(R_1\ R_2\ R_3\ R_4)\ P]^+\ X^-,\qquad (1)$$

wobei $R_1$, $R_2$, $R_3$ und $R_4$ gleichartige oder verschiedene Alkyl- oder Arylgruppen bedeuten. Alkylgruppen können solche mit 1 bis 4 C-Atomen, also Methyl-, Ethyl-, n- und i-Propyl-, 2-Methyl-3-propyl, 3-Methyl-3-propyl sowie n- und i-Butylgruppen sein. Arylgruppen können Phenyl-, Benzyl oder entsprechende substituierte monocyclische Aromaten sein. X kann Chlor, Brom oder Jod sein. Aber auch andere Salze der erfindungsgemäßen quartären Phosphoniumionen können als Katalysator eingesetzt werden.

Beispiele für die erfindungsgemäß zu verwendenden Katalysatoren sind Ethyltriphenylphosphoniumbromid, Tetrabutylphosphoniumbromid, Tetraphenylphosphoniumchlorid, Butyltriphenylphosphoniumchlorid, 4-Ethoxybenzyltriphenylphosphoniumbromid und Methoxymethyltriphenylphosphoniumchlorid. Diese Verbindungen können nach in E. Müller, Methoden der organischen Chemie, Bd. XII/1, 4. Aufl. 1963, S. 79 ff, G. Thieme Verlag Stuttgart beschriebenen Verfahren hergestellt werden.

Diese Phosphoniumverbindungen werden im allgemeinen in einer Menge von 0,05 bis 10 %, vorzugsweise von 0,1 bis 5 % bezogen auf das Gewicht der Epoxidkomponente eingesetzt.

Die Herstellung von 2-Oxo-1,3-dioxolanen aus Glycidylverbindungen und Kohlendioxid kann in An- oder Abwesenheit eines inerten Lösungsmittels geschehen. Falls die Viskosität der Glycidylverbindung oder des entstehenden 2-Oxo-1,3-dioxolans bei der Reaktionstemperatur niedrig ist, wird normalerweise kein Lösungsmittel verwendet. Liegt das Reaktionsgemisch als viskose Schmelze vor oder ist eine Weiterverarbeitung in Lösung vorgesehen, empfiehlt sich die Verwendung eines inerten Lösungsmittels wie z. B. Toluol, Xylol, Tetrahydrofuran, Diethylenglycoldimethylether oder Dimethylsulfoxid. In diesem Fall wird die Epoxidkomponente im 0,05- bis 5-fachen ihres Volumens, vorzugsweise im 0,2- bis 2-fachen ihres Volumens in einem Lösungsmittel gelöst und in Gegenwart eines erfindungsgemäßen Katalysators umgesetzt.

In dem erfindungsgemäßen Verfahren können Glycidylverbindungen eingesetzt werden, die mindestens eine Epoxidgruppe im Molekül enthalten. Hierzu zählen im "Handbook of Epoxy Resins" (Lee, H.; Neville, K.; Mc Graw Hill Book Company, New York, 1967) genannte Epoxidverbindungen, wie Glycidyl-ether, -amine, -polyamine oder -ester.

Die Glycidylverbindungen können mit $CO_2$ in Gegenwart eines erfindungsgemäßen Katalysators als solche oder gelöst in einem Lösungsmittel der Gruppe Dimethylsulfoxid, Diethylenglycoldimethylether oder Toluol, bei Temperaturen von 60 bis 200 °C und Normaldruck umgesetzt werden. Vorzugsweise wird bei Temperaturen von 80 bis 160 °C gearbeitet.

Je nach Reaktivität der umzusetzenden Glycidylverbindung, der eingestellten Temperatur, des gewählten Katalysators, evtl. des Lösungsmittels und des gewünschtem Endprodukts ergeben sich unterschiedliche Reaktionszeiten.

Unter den genannten, bevorzugten Bedingungen hat nach 5 bis 10 h eine nahezu vollständige Umsetzung der Epoxidgruppen zu 2-Oxo-1,3-dioxolanen stattgefunden. Der Restgehalt an Epoxidgruppen kann auf an sich bekannte Weise durch Titration mit Perchlorsäure ermittelt werden. Soll ein Produkt hergestellt werden, in dem noch ein gewisser Teil funktioneller Epoxygruppen enthalten ist, kann die Reaktion jeder Zeit beendet werden.

Durch die Verwendung der erfindungsgemäßen quartären Phosphoniumsalze als Katalysatoren für Reaktionen von Glycidylverbindungen mit Kohlendioxid zu cyclischen Carbonaten wird eine erhebliche

3

Steigerung der Reaktionsgeschwindigkeit erzielt. Bei Normaldruck und unter Verzicht auf jegliche Cokatalysatoren kann die Reaktionsgeschwindigkeit im Vergleich zu anderen Verfahren um bis zu 150 % gesteigert werden.

Es ist ein weiterer Vorteil der erfindungsgemäß zu verwendenden Katalysatoren, daß sie sich als recht inert gegenüber Reaktionen mit dem eingeleiteten $CO_2$ erweisen, so daß die Katalysatoraktivität bis zum Schluß der Reaktion nahezu vollständig erhalten bleibt. Auch treten praktisch keine Epoxid-Nebenreaktionen wie Homopolymerisationen auf.

Von besonderem Vorteil ist die gute Löslichkeit der farblosen Katalysatoren in den Reaktionsprodukten, so daß sogar ohne Abtrennung gute Farbqualitäten erhalten werden. Während z. B. gereinigte Reaktionsprodukte, die nach dem in EP-A 212 409 beschriebenen Verfahren mit Ammoniumsalz-Katalysatoren hergestellt wurden, Farbzahlen von 12 bis 15 nach Gardner aufweisen, wurden nach dem erfindungsgemäßen Verfahrenen cyclische Carbonate erhalten, die im Rohzustand bereits Werte von etwa 2 bis 5 aufwiesen. Cyclische Carbonate aus Glycidylaminen weisen, bedingt durch die höheren Farbzahlen der Ausgangs-Epoxidharze und ihre geringe thermische Stabilität, höhere Farbzahlen auf.

Vorteilhaft ist weiterhin die hohe Selektivität dieser Katalysatoren, da auf diese Weise im erfindungsgemäßen Verfahren einerseits Produkte mit hoher Reinheit erhalten werden, und andererseits aber auch nur sehr geringe Katalysatormengen eingesetzt werden müssen. Das ist von besonderer Bedeutung, wenn die Reaktionsprodukte im Bereich der Elektrik oder Elektronik verwendet werden sollen, da sie in diesem Fall nur einen minimalen Elektrolytgehalt aufweisen dürfen.

Eine funktionelle Wirkung des im Produkt verbleibenden Katalysators (z. B. Weichmachung, Stabilisierung) konnte nicht nachgewiesen werden.

Zur Durchführung des Verfahrens wird die evtl. gelöste Glycidylverbindung in einem mit Thermometer, Rührer, Gasein- und ableitungsrohr ausgestatteten Reaktionsgefäß vorgelegt und der Phosphoniumsalz-Katalysator hinzugefügt. Bei Erwärmung auf Reaktionstemperatur geht der Katalysator in Lösung. Unter kräftigem Rühren wird so schnell wie möglich Kohlendioxid eingeleitet, ohne daß nicht abreagiertes, überschüssiges Gas durch das Gasableitungsrohr entweicht. Alles $CO_2$ wird spontan umgesetzt, so daß sich kein nennenswerter Überdruck aufbaut.

Die Umsetzung wird bis zum gewünschten, durch Titration bestimmten Rest-Epoxidgehalt fortgesetzt. Evtl. vorhandenes Lösungsmittel wird bei vermindertem Durck abdestilliert.

Die nachfolgenden Beispiele und Tabellen dienen zur weiteren Erläuterung, ohne jedoch auf diese zu begrenzen.

**Beispiele**

Beispiel 1

In einem Reaktionsgefäß, das mit Thermometer, Rührer, Gasein- und ableitungsrohr ausgestattet ist, werden 186 g des Epoxidharzes VE 0162 vorgelegt und mit 2,1 g Ethyltriphenylphosphoniumbromid versetzt. Unter Rühren wird das Gemisch auf eine Temperatur von 140 °C erhitzt, wobei sich der Katalysator auflöst. Die Rührgeschwindigkeit wird nun erhöht und Kohlendioxid in feinen Gasblasen derart eingeleitet, daß das eingeleitete Gas vollständig durch die Reaktion verbraucht wird.Durch mehrfache Probenahme und Bestimmung des Restgehalts an Epoxidgruppen durch Titration mit Perchlorsäure nach einer in DIN 16945 beschriebenen Methode wird der Reaktionsverlauf kontrolliert. (Bei glycidylierten Aminen wird die in H. Lee, K. Neville, Handbook of Epoxy Resins, Mc Graw Hill New York, 1976, S. 4 bis 17 geschilderte Pyridin/Salzsäure-Methode angewendet.)

Es entsteht ein reines und daher hochschmelzendes kristallisationsfähiges Produkt (Schmelztemperatur = 150 °C), das zum Ende der bei 140 °C ablaufenden Reaktion als kristalliner Feststoff ausfällt. Zur Vervollständigung der Reaktion wird daher die Temperatur auf ca. 160 °C erhöht.

Nach 9 h ist der Gehalt an funktionellen Epoxygruppen auf 0,6 % gesunken, und die Reaktion wird beendet. Das erhaltene Produkt zeigte folgende Eigenschaften:

Schmelzpunkt: 150 °C

Farbzahl: 2     ermittelt nach Gardner

Ergebnisse weiterer Umsetzungen, die in gleicher Weise durchgeführt wurden, sind in Tabelle 1 zusammengefaßt, Darin bedeuten die Abkürzungen:

VE 0162     Bisphenol A-diglycidylether, destillierte Qualität

VE 0164     Bisphenol A-diglycidylether, technische Qualität

VE 0161     Bisphenol F-diglycidylether

VE 0300     Epoxidierter Phenol-Novolak

| VE 4162 | Epoxidiertes Pentaerythrit |
|---|---|
| VE 3650 | N,N-Diglycidylanilin |
| VE 2895 LV | Tetraglycidylmethylendianilin |
| CY 160 | Hexahydrophthalsäurediglycidylester |
| Verdünner S | Hexandioldiglycidylether |
| Verdünner T | Trimethylolpropanglycidylether |

Vergleichsbeispiel

Vergleichsweise wird das Harz des Beispiels 1, VE 0162, gemäß der allgemeinen Vorschrift zur Herstellung von Bicarbonaten aus Diglycidylethern des Bisphenol A aus EP-A 212 409 mit Tetraethyammoniumbromid als Katalysator und KJ als Co-Katalysator umgesetzt (Tabelle 1 Nr. 13).

Es entsteht ein orange gefärbtes weniger reines und deshalb nicht kristallisationsfähiges cyclisches Carbonat, das bei Abkühlung auf Raumtemperatur glasig erstarrt unf eine Erweichungstemperatur von 51 bis 52 °C aufweist.

EP 0 477 472 B1

Tabelle 1

| Nr. | EP-Harz | EP-Zahl % | Menge Teile | Katalysa- tor-Teile | Katalysa- tor % | Lösungs- mittel | Lösungs- mittel Teile | Temperatur °C |
|---|---|---|---|---|---|---|---|---|
| 1 | VE 0162 | 9,3 | 1.860 | 21 | 1,12 | ------- | ------- | 140 - 160 |
| 2 | VE 0164 | 8,6 | 2.670 | 27 | 1,01 | ------- | ------- | 140 |
| 3 | VE 0164 | 8,6 | 2.940 | 31 | 1,05 | ------- | ------- | 60 |
| 4 | VE 0164 | 8,6 | 3.060 | 32 | 1,05 | ------- | ------- | 200 |
| 5 | VE 0161 | 9,2 | 2.810 | 30 | 1,07 | ------- | ------- | 140 |
| 6 | VE 0300 | 9,1 | 2.950 | 30 | 1,02 | ------- | ------- | 160 |
| 7 | Verdünner S | 11,2 | 1.500 | 16 | 1,06 | ------- | ------- | 100 |
| 8 | Verdünner T | 10,9 | 2.830 | 29 | 1,02 | ------- | ------- | 100 |
| •9 | VE 4162 | 9,9 | 2.814 | 30 | 1,07 | ------- | ------- | 120 |
| 10 | CY 160 | 9,5 | 3.096 | 31 | 1,02 | ------- | ------- | 120 |
| 11 | VE 2895 LV | 13,8 | 2.800 | 29 | 1,03 | Toluol | 2.000 | 100 |
| 12 | VE 3650 | 13,7 | 2.000 | 21 | 1,05 | ------- | ------- | 100 - 120 |
| 13 | Vergleichs- versuch VE 0162 | 9,3 | 1.860 | 21 | 1,12 + 20 Teile Cokataly- sator KJ (1,1 %) | ------- | ------- | 140 |
| 14 | VE 0164 | 8,6 | 2.961 | 2,7 | 0,09 | ------- | ------- | 140 |
| 15 | VE 0164 | 8,6 | 3.049 | 98 | 3.21 | ------- | ------- | 140 |

Tabelle 1 (Fortsetzung)

| Nr. | Reaktions-zeit h | Ausbeute Teile | Ausbeute % | Rest-EP-Gehalt % | Schmelz-punkt °C | Farbzahl (Gardner) EP-Harz | Farbzahl (Gardner) Cycl. Cabonat |
|---|---|---|---|---|---|---|---|
| 1 | 9 | 2.234 | 95,6 | 0,2 | 150 | 1 | 2 |
| 2 | 6 1/2 | 3.206 | 97,2 | 0,1 | 49 | 2 | 2 |
| 3 | 18 | 3.210 | *) | 2,2 | (hoch viskos) | 2 | 2 |
| 4 | 7 | 3.502 | *) | 1,5 | (fast fest) | 2 | 12 |
| 5 | 8 1/2 | 3.415 | 96,8 | 0,2 | 41 | 4 | 5 |
| 6 | 14 | 3.460 | 94,1 | 0,2 | 76 | 5 | 5 |
| 7 | 10 | 1.911 | 97,3 | 0,2 | (flüssig) | 1 | 2 |
| 8 | 10 | 3.516 | 95,5 | 0,2 | (flüssig) | 1 | 2 |
| 9 | 8 | 3.501 | 97,9 | 0,2 | (hoch viskos) | 2 | 3 |
| 10 | 5 | 3.780 | 96,8 | 0,1 | (fast fest) | 2 | 4 |
| 11 | 10 | 3.692 | 95,5 | 0,4 | 74 | 10 | 17 |
| 12 | 9 | 2.612 | 94,7 | 0,3 | (fast fest) | 4 | 14 |
| 13 | Vergleichs-versuch 19 | 2.238 | 95,7 | 0,2 | 51 - 52 | 1 | 13 - 14 |
| 14 | 15 | 3.549 | 97,9 | 0,3 | 48 | 2 | 3 |
| 15 | 3 1/2 | 3.670 | 97,4 | 0,1 | 50 - 51 | 2 | 3 |

*) Teilweise Umsetzung der EP-Gruppen

## Patentansprüche

1. Verfahren zur Herstellung von 2-Oxo-1,3-dioxolanen durch Umsetzung einer Glycidylverbindung ausgewählt aus Glycidylethern, glycidylierten Aminen, glycidylierten Polyaminen und Glycidylestern, dadurch

7

gekennzeichnet, daß die Glycidylverbindung, die mindestens eine funktionelle Epoxygruppe besitzt, als solche oder gelöst in einem inerten Lösungsmittel in einem offenen System bei Temperaturen von 60 bis 200 °C durch Einleiten von Kohlendioxid umgesetzt wird, wobei eine quartäre Phosphoniumverbindung der Formel

$$[(R_1 R_2 R_3 R_4)P]+ \ X-$$

wobei es sich bei $R_1$, $R_2$, $R_3$, und $R_4$ um gleiche oder verschiedene Alkylsubstituenten mit 1 bis 4 C-Atomen oder substituierte oder nicht substituierte Phenyl- oder Benzylreste handelt, und X ein Halogen wie Chlor, Brom, Jod oder ein anderes Anion ist, als Katalysator eingesetzt wird.

2. Verfahren nach Anspruch 1, dadurch gekennzeichnet, daß der Katalysator in einer Konzentration von 0,05 bis 10 Gew.-% bezogen auf die Menge der Glycidylverbindung eingesetzt wird.

3. Verfahren nach Anspruch 1, dadurch gekennzeichnet, daß der Katalysator in einer Konzentration von 0,1 bis 5 Gew.-% bezogen auf die eingesetzte Menge Glycidylverbindung eingesetzt wird.

4. Verfahren nach den Ansprüchen 1 bis 3, dadurch gekennzeichnet, daß die umzusetzende Glycidylverbindung mindestens eine endständige funktionelle Epoxygruppe besitzt.

5. Verfahren nach den Ansprüchen 1 bis 4, dadurch gekennzeichnet, daß die Umsetzung der Epoxygruppen nicht vollständig erfolgt.

6. Verfahren nach den Ansprüchen 1 bis 5, dadurch gekennzeichnet, daß die Umsetzung in Gegenwart einer quartären Phosphoniumverbindung aus der Gruppe Ethyltriphenylphosphoniumbromid, Tetrabutyl-phosphoniumbromid, Tetraphenylphosphoniumchlorid, Butyltriphenylphosphoniumchlorid, 4-Ethoxyben-zyltriphenylphosphoniumbromid und Methoxymethyltriphenylphosphoniumchlorid als Katalysator stattfindet.

7. Verfahren nach den Ansprüchen 1 bis 5, dadurch gekennzeichnet, daß die Umsetzung in Gegenwart einer quartären Phosphoniumverbindung der Formel

$$[(R_1 R_2 R_3 R_4)P]+ \ X-$$

wobei es sich bei $R_1$, $R_2$, $R_3$, und $R_4$ um gleiche oder verschiedene Reste ausgewählt aus 2-Methyl-3-propyl-, 3-Methyl-3-propyl-, Phenyl-, Benzyl- und Tolylgruppen handelt,
und X ein Halogen wie Chlor, Brom, Jod oder ein anderes Anion ist.

**Claims**

1. A process for preparing 2-oxo-1,3-dioxolanes by reacting a glycidyl compound of the group from glycidyl ethers, glycidyl amines, glycidyl polyamines and glycidyl esters, **characterized in that** the glycidyl compound having at least one functional epoxy group is reacted as such or dissolved in an inert solvent in an open system at temperatures of from 60 to 200 °C by feeding carbon dioxide, whereas a quaternary phosphonium compound having the formula

$$[(R_1 R_2 R_3 R_4)P]^+ X^-$$

is used as catalyst,
wherein $R_1$, $R_2$, $R_3$ and $R_4$ are equal or different alkyl substituents with 1 to 4 carbon atoms or substituted or unsubstituted phenyl or benzyl radicals and X is an halogen like chlorine, bromine or iodine or another anion.

2. A process according to claim 1, **characterized in that** the catalyst is introduced in a concentration of from 0,05 to 10 % by weight relative to the quantity of the glycidyl compound.

3. A process according to claim 1, **characterized in that** the catalyst is introduced in a concentration of from 0,1 to 5 % by weight relative to the glycidyl compound.

4. A process according to claims 1 to 3, **characterized in that** the glycidyl compound to be reacted has at least one functional epoxy group at the end.

5. A process according to claims 1 to 4, **characterized in that** the reaction of the epoxy groups does not take place completely.

6. A process according to claims 1 to 5, **characterized in that** the reaction takes places in the presence of a quaternary phosphonium compound of the group from ethyl-triphenylphosphonium bromide, tetrabutyl-phosphonium chloride, butyltriphenyl-phosphonium bromide, 4-ethoxybenzyl-triphenylphosphonium bromide and methoxymethyl-triphenylphosphonium chloride.

7. A process according to claims 1 to 5, **characterized in that** the reaction takes place in the presence of a quaternary phosphonium compound of the formula

$$[(R_1 R_2 R_3 R_4)P]^+ X^-$$

wherein $R_1$, $R_2$, $R_3$ and $R_4$ are equal or different radicals chosen from the 2-methyl-3-propyl- or 3-methyl-3-propylphenyl, benzyl- or tolyl-group and X is a halogen like chlorine, bromine, iodine or another anion.

**Revendications**

1. Procédé de préparation de 2-oxo-1,3-dioxolanes par réaction d'un composé glycidique choisi du groupe des éthers glycidiques, des amines glycidylées, des polyamines glycidylées ou des esters glycidiques, **caractérisé en ce que** le composé glycidique possédant au moins un groupe époxy fonctionnel est mis à réagir, en tant que tel ou en solution dans un solvant inerte dans un système ouverte à des temperatures entre 60 à 200 °C par introduction de dioxide carbone, un composé de phosphonium quaternaire de formule

$$[(R_1 R_2 R_3 R_4)P]^+ X^-$$

est introduit
auquel cas il s'agit, à propos de $R_1$, $R_2$, $R_3$ et $R_4$, de substituants alkyles indentiques ou différents avec 1 à 4 atomes de C ou des restes phényle ou benzyle substitués ou non substitués, et que X est une halogène tel que le chlore, le brome, l'iode ou un autre anion.

2. Procédé selon la renvendication 1, **caractérisé en ce que** le catalyseur est mis en oeuvre à une concentration de 0,05 à 10% en poids par rapport à la quantité de composé glycidique.

3. Procédé selon la revendication 1, **caractérisé en ce que** le catalyseur est mis en oeuvre à une concentration de 0,1 à 5% en poids par rapport à la quantité de composé glycidique.

4. Procédé selon les revendications 1 à 3, **caractérisé en ce que** le composé glycidique à faire réagir possède au moins un groupe époxy fonctionnel terminal.

5. Procédé selon les revendications 1 à 4, **caractérisé en ce que** la réaction des groupes époxy n'a pas lieu totalement.

6. Procédé selon les revendications 1 à 5, **caractérisé en ce que** la réaction est fait en présence d'un composé de phosphonium quaternaire comme catalyseur, le composé de phosphonium quaternaire étant du groupe éthyltriphenylphosphonium bromide, tetrabutylphosphonium bromide, tetraphénylphosphoniumchloride, butyltriphénylphophoniumchloride, 4-éthoxybenzyltriphénylphosphoniumbromide ou methoxymethyltriphénylphosphoniumchloride.

7. Procédé selon les revendications 1 à 5, **caractérisé en ce que** la réaction est fait en présence d'un composé de phosphonium quatenaire de formule

$$[(R_1 R_2 R_3 R_4)P]^+ X^-$$

**EP 0 477 472 B1**

auquel cas is s'agit, à propos de $R_1$, $R_2$, $R_3$ et $R_4$ de substituants identiques ou différents choisi de radicals 2-methyl-3-propyl-, 3-methyl-3-propyl-, phényl-, benzyl et tolyl- et X est un halogène tel que le chlore, le brome, l'iode ou un autre anion.

10